(19) Europäisches Patentamt
European Patent Office
Office européen des brevets

(11) Publication number: 0 453 692 A1

# (12) EUROPEAN PATENT APPLICATION

(21) Application number: 90401169.9

(22) Date of filing: 27.04.90

(51) Int. Cl.5: C07F 7/08, A61K 31/695, C07F 7/18

(43) Date of publication of application: 30.10.91 Bulletin 91/44

(84) Designated Contracting States: FR

(71) Applicant: MERRELL DOW PHARMACEUTICALS INC.
P.O. Box 156300 2110 East Galbraith Road
Cincinnati Ohio 45215-6300(US)

(72) Inventor: Lesur, Brigitte
3 Rue Klein
F-67000 Strasbourg(FR)
Inventor: Ducep, Jean-Bernard
29 Rue des Alpes
F-68280 Sundhoffen(FR)
Inventor: Danzin, Charles
18 Rue Geiler
F-67000 Strasbourg(FR)

(74) Representative: Gillard, Marie-Louise et al
Cabinet Beau de Loménie 55, Rue d'Amsterdam
F-75008 Paris(FR)

## (54) Novel nojirimycin derivatives.

(57) This invention relates to novel N-derivatives of 1-deoxy nojirimycin, to the method for their preparation and to their use in the treatment of diabetes and the use against retro-viruses, particularly in the treatment of acquired immuno-deficiency syndrome (AIDS).

EP 0 453 692 A1

This invention relates to novel N-derivatives of 1-deoxy nojirimycin, to the method for their preparation and to their use in the treatment of diabetes and the use against retro-viruses, particularly in the treatment of acquired immuno-deficiency syndrome (AIDS).

More specifically the invention relates to 1-deoxy nojirimycin derivatives of the formula

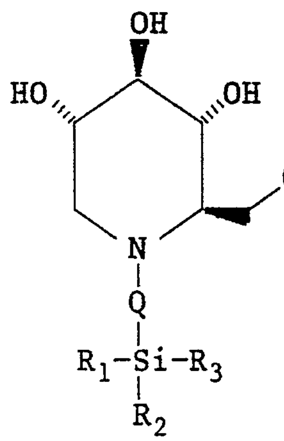

I

and the pharmaceutically acceptable salts thereof wherein

Q is $C_{1-7}$ alkylene, $-(CH_2)_m-HC=CH-(CH_2)_n$, $-(CH_2)_mC{\equiv}C-(CH_2)_n$,$(CH_2)_mCH=C=CH(CH_2)_n$ with m being 1 or 2 and n being zero, 1 or 2,

$R_1$ is $C_{1-7}$ alkyl or $C_{1-7}$ alkoxy, each of

$R_2$ and $R_3$ are $C_{1-10}$ alkyl or $-(CH_2)_p$-X,Y-substituted phenyl, with X and Y each being H, OH, halogeno, $C_{1-6}$ alkyl, $C_{1-6}$ alkoxy, $CF_3$, CN, $NO_2$, SH or $-S-C_{1-6}$ alkyl, and p is an integer 1 to 4 or zero.

As used herein, Q is a divalent bridging moiety having up to seven carbon atoms separating the 1-deoxy nojirimycin moiety from the $-SiR_1R_2R_3$ moiety. The $C_{1-7}$ alkylene moiety includes the straight and cyclized manifestations thereof, particularly such alkylene moieties derived from alkyl radicals as methyl, ethyl, propyl, n-butyl, cyclopropyl, pentyl, n-hexyl, cyclohexyl and cyclohexylmethyl, preferably n-butyl and n-propyl. The $-(CH_2)_p$-X,Y-substituted phenyl moiety includes X,Y substituted phenyl, benzyl, phenethyl, phenylpropyl and phenylbutyl with benzyl and phenethyl being preferred. The term halogeno includes all four members with fluoro being preferred. Preferably, at least one of X and Y is hydrogen, but the generic scope still includes the mono and di-substituted phenyl moiety including the 2-,3- and 4-mono and 2,4-, 2,6-, 3,5-and 3,4-di-substituted phenyl, benzyl, phenethyl and the like moieties. Preferably m is one and n is zero or one. Preferred alkyl moieties are methyl and ethyl, preferred alkoxy are methoxy and ethoxy and when X or Y represents an alkylthiol, methylthiol is preferred. For convenience, the

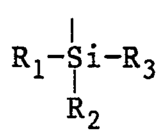

moiety and the

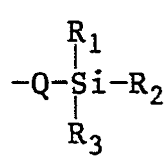

moiety of Formula I will also be referred to as $-SiR_1R_2R_3$ and $-Q-Si-R_2R_2R_3$, respectively.

The pharmaceutically acceptable salts of the compounds of formula I include salts formed with non-toxic organic or Inorganic acids such as, for example, from the following acids:hydrochloric, hydrobromic, sulfonic, sulfuric, phosphoric, nitric, maleic, fumaric, benzoic, ascorbic, pamoic, succinic, methanesulfonic acid, acetic, propionic, tartaric, citric, lactic, malic, mandelic, cinnamic, palmitic, itaconic and benzenesulfonic and the like.

In general the compounds of this invention are prepared by chemical reactions and procedures which are analogously known in the art and the selection of a particular route to any specific compound is governed by principles well known and appreciated by those of ordinary skill in the art.

In general the compounds of this invention may be prepared according to the reaction scheme outlined below.

Reaction Scheme A

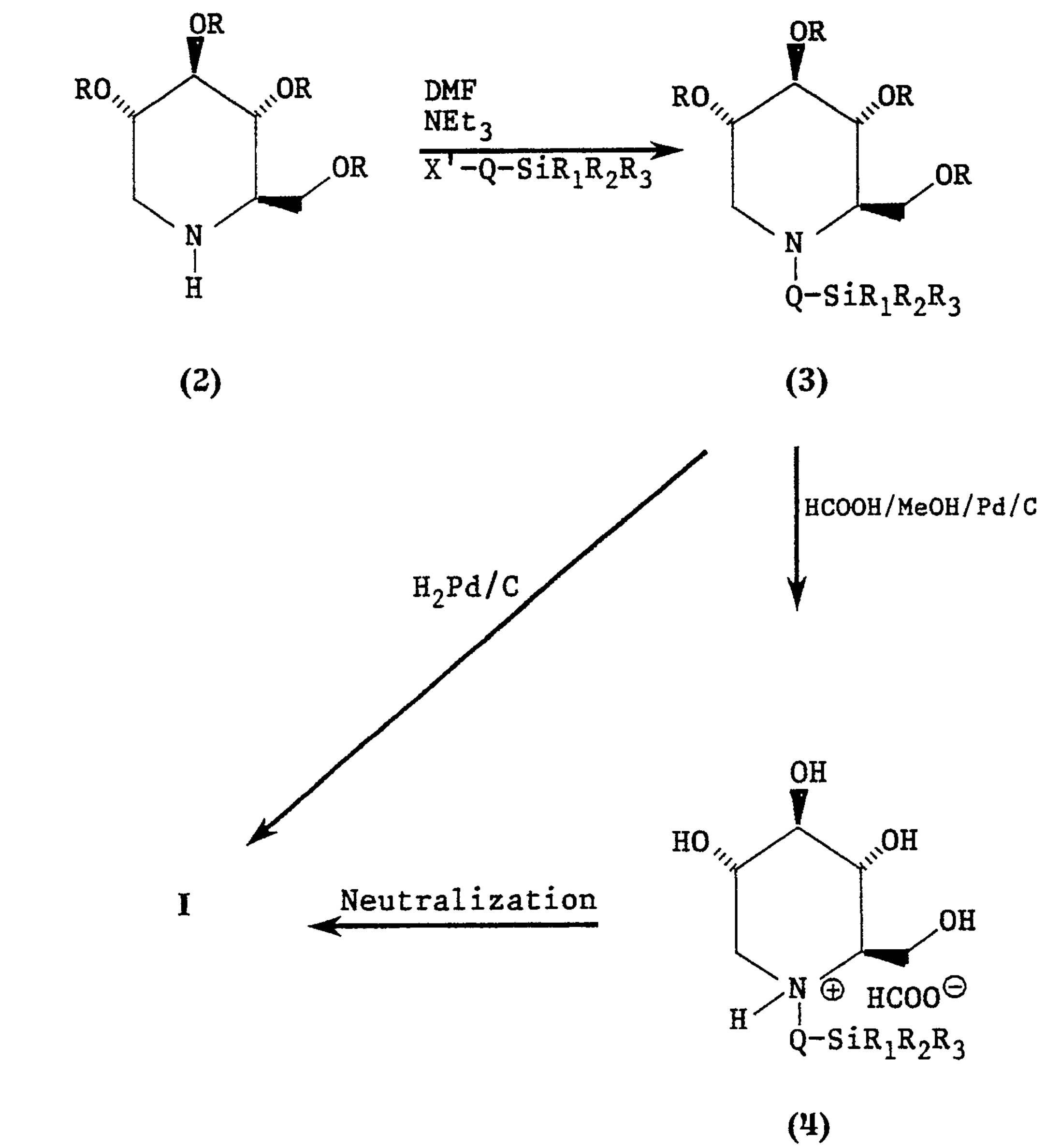


(2) (3) (4) I

wherein $Q\text{-}SiR_1R_2R_3$ is as previously defined, R is H or Bz, Bz is benzyl, a preferred hydroxy-protecting group, X' is halogeno, mesylate or tosylate.

The synthesis of Reaction Scheme A is initiated by the condensation of an optionally hydroxy-protected 1-deoxy nojirimycin with an excess quantity (≃ three times) of the appropriate $X'\text{-}Q\text{-}SiR_1R_2R_3$ reactant in the presence of an excess of triethylamine ($NEt_3$) in dimethyl formamide (DMF). Preferably X' is the iodide. The so-produced compounds (3) are purified, preferably using chromatographic techniques and are then deprotected according to standard procedures well known in the art. Preferably deprotection is effected by using transfer hydrogenation with formic acid in methanol with Pd/C or by catalytic hydrogenation, preferably using palladium on carbon in an appropriate solvent, e.g. ethanol. When transfer hydrogenation is

utilized, the deprotected products (4) are in the form of quaternary salts with the $HCOO^{\ominus}$ anion and thus must be neutralized; the neutralization preferably being effected using an ion exchange resin such as Dowex AX-8.

In those instances wherein Q represents a bridging moiety containing an unsaturation (i.e., allylic, allenic or acetylenic) it is preferred that the 2-, 3- and 6-position hydroxy groups not be protected in view of the difficulties encountered when removing the benzyl protecting groups unless special procedures are employed (e.g., when Q contains allylic or allenic moieties the use of sodium in ammonia). In those instances wherein Q is other than a moiety containing an allylic, allenic or acetylenic moiety, it is preferred that the 2-, 3- and 6-positions bear a benzyl protecting group for "normal" procedures (as outlined above) may be employed for their removal, and the benzyl groups also serve as an excellent means for obtaining the products in a more purified form than when the free-hydroxy compounds are utilized.

1-Deoxy nojirimycin may be obtained by reducing nojirimycin (5-amino-5-deoxy-D-glucopyranose) using the method of Tetrahedron Letters, 24, 2125-2144, 1968, as referenced in European Patent Application 89 112284.8 published on January 10, 1990, with publication No. 0350012. The preparation of 1-deoxy nojirimycin and its hydroxy-protected analogs (2) are also disclosed in the specific examples disclosed herein.

The $X'$-Q-$SiR_1R_2R_3$ reactant are either known compounds or may be prepared by methods analogously known in the art.

The following examples illustrate the preparation of the compounds of this invention.

## EXAMPLE 1

### Preparation of 3-(TRIMETHYLSILYL)-1-PROPANOL, METHANESULFONATE

Methanesulfonylchloride (0.73 ml, 9 mmol) was added dropwise to a solution of 3-(trimethylsilyl)-1-propanol (1.2 ml, 7.56 mmol) cooled at 0 °C in 20 ml of dichloromethane. After 45 minutes stirring, the reaction mixture was partitioned between water and dichloromethane, the organic phase was separated, the solvent was evaporated under reduced pressure to afford the expected 3-(trimethylsilyl)-1-propanol, methanesulfonate in crude quantitative yield.

## EXAMPLE 2

### Preparation of (3-IODOPROPYL)TRIMETHYSILANE

An ethereal solution of magnesium iodide was prepared by adding 4.85 g (19 mmol) of iodine to 0.46 g (19 mmol) of magnesium in suspension in 40 ml of dry ether. 28 ml of this solution was added to a solution containing the crude 3-(trimethylsilyl)-1-propanol, methanesulfonate in 10 ml of ether. The reaction mixture was stirred for 3 hours at room temperature and then partitioned between ether and water, the organic phase was separated and further washed with aqueous sodium thiosulfate. After evaporation of the solvent, 1.6 g of (3-iodopropyl)trimethysilane was obtained as a colorless liquid.

## EXAMPLE 3

### Preparation of (3-BROMOPROPYL )TRIMETHYLSILANE

1.02 ml (10.9 mmol) of phosphorous tribromide in 20 ml of ether was added to a cooled (0 °C, -10 °C) solution of 4 g (30.2 mmol) of 3-(trimethylsilyl)-1-propanol in 40 ml of dry ether. The reaction mixture was brought back to room temperature and refluxed for 15 minutes. After bulb to bulb distillation of the crude reaction mixture 4.3 g of (3-bromopropyl)trimethylsilane were isolated as a colorless liquid.

## EXAMPLE 4

### Preparation of 4-(TRIMETHYLSILYL)-BUTANOIC ACID

A solution containing 3 g (15.4 mmol) of (3-bromopropyl)trimethylsilane in 3 ml of dry ether was added to 0.375 g (15.4 mmol) of magnesium turnings in ether (40 ml final volume of solution). After 1 hour of reflux gazeous carbondioxide was bubbled through the reaction mixture (3 g, 77 mmol of dry ice). After 2 hours of stirring at room temperature the reaction mixture was partitioned between aqueous ammonium chloride and ether. The organic phase was separated, the aqueous phase was further acidified with hydrochloric acid 1N and extracted with ether. Ethereal phases were pooled and the solvent was removed under reduced pressure. Separation of the expected 4-trimethylsilylbutanoic acid from dimer of starting (3-bromopropyl)-trimethylsilane was finally performed by acid base extraction. 0.62 g of 3-(trimethylsilyl)-butanoic acid was isolated as a colorless liquid.

EXAMPLE 5

Preparation of
4-(TRIMETHYLSILYL)-1-BUTANOL

1.5 ml (11.4 mmol) of a 1 molar solution of borane dimethylsulfide was added to a cooled (0°C) solution of 0.61 g (3.8 mmol) of 4-(trimethylsilyl)-butanoic acid in 20 ml of dry tetrahydrofuran. After work-up using the standard procedure (methanol, tetramethylethylene diamine) and flash chromatography purification on silica gel eluted with a 9:1 mixture of hexane and ethyl acetate, 0.4 g of 4-(trimethylsilyl)-1-butanol was obtained as a colorless liquid.

EXAMPLE 6

Preparation of
4-(TRIMETHYLSILYL)-1-BUTANOL, METHANESULFONATE

Starting from 0.4 g (2.53 mmol) of 4-(trimethylsilyl)-1-butanol, 0.245 ml (3.16 mmol) of methanesulfonyl chloride and 0.528 ml (4.3 mmol) of triethylamine, and using the same procedure as for the preparation of 3-(trimethylsilyl)-1-propanol, methanesulfonate, 0.5 g of the expected 4-(trimethylsilyl)-1-butanol, methanesulfonate was obtained.

EXAMPLE 7

Preparation of
4-(IODOBUTYL)TRIMETHYLSILANE

Using the same procedure as described for the preparation of 3-(iodopropyl)trimethylsilane, starting from 0.5 g (2.53 mmol) of 4-(trimethylsilyl)-1-butanol methane sulfonate and 12 ml of a 0.34 M ethereal solution of magnesium iodide, 0.5 g of 4-(iodobutyl)trimethylsilane was isolated as a colorless liquid.

EXAMPLE 8

Preparation of
(4-BROMO-2-BUTYNYL)TRIMETHYLSILANE

4-(trimethylsilyl)-2-butynol [J. Pernet, B. Randrianoelina, and L. Miginiac, Tetrahedron Letters, 25, 651, (1984)] (10 g, 70 mmol) is dissolved in dry diethyl ether (150 ml) and phosphorous tribromide (2.2 ml, 23.3 mmol) is added dropwise. Then the mixture is refluxed, protected from the light during 2.5 hours. The reaction is washed twice with water, once with aqueous sodium bicarbonate and then once with water. The organic layer is dried over sodium sulfate. The solvent is evaporated under reduced pressure to afford the expected bromide (4-bromo-2-butynyl)trimethylsilane (1.4 g, 97%) which is used without purification.

EXAMPLE 9

Preparation of
(4-BROMO-2-(E)BUTENYL)TRIMETHYLSILANE

4-(Trimethylsilyl)-2(E)-butene-1-ol [H. Mastalerz, J. Org. Chem., 49, 4094, (1984)] (10 g, 70 mmol) is

dissolved in dry diethyl ether (150 ml) and phosphorous tribromide (2.2 ml, 23.3 mmol) is added dropwise. Then the mixture is refluxed, protected from the light during 2.5 hours. The reaction is washed twice with water, once with aqueous sodium bicarbonate and then once with water. The organic layer is dried over sodium sulfate. The solvent is evaporated under reduced pressure to afford the expected bromide (4-bromo-2-(E)butenyl)trimethylsilane (1.4 g, 97%) which is used without purification.

EXAMPLE 10

Preparation of
DIMETHYL(3-IODOPROPYL)PHENYLSILANE

Dimethyl(3-chloropropyl)phenylsilane [J.W. Wilt, W.K. Chwang, C.F. Dockus and N.M. Tomiuk, J. Am. Chem. Soc., 100, 5534, (1978)] (9 g, 48.8 mmol) and sodium iodide (29.5 g, 195 mmol) are refluxed in acetone during 24 hours. The mixture is filtered and the solvent is evaporated under reduced pressure. The residue is dissolved in diethyl ether and washed with water. The organic layer is dried with sodium sulfate, filtered and concentrated under reduced pressure to afford pure dimethyl(3-iodopropyl)phenylsilane as a slightly yellow oil (12.5 g, 93%).

EXAMPLE 11

Preparation of
BENZYL(IODOMETHYL)DIMETHYLSILANE

Benzyl(bromomethyl)dimethylsilane [Colm, Earborn and Foad M.S., Malmond J. Organomet. Chem., 209, 13 (1981)] (12 g, 0.05 mmol) and sodium iodide (45 g, 0.3 mmol) are refluxed with stirring in acetone (500 ml) during 24 hours. The reaction mixture is cooled, filtered and the solvent is evaporated under reduced pressure. The residue is dissolved in ether and washed with water. The organic layer is dried over sodium sulfate, filtered and concentrated under reduced pressure to afford benzyl(iodomethyl)dimethylsilane (13.7 g, 95%) as a slightly yellow oil.

EXAMPLE 12

Preparation of
t-BUTYL(IODOMETHYL)DIMETHYLSILANE

t-Butyl(chloromethyl)dimethylsilane [Makoto Kumada, Mitsuo Ishikawa, Sajiro Meada and Katsuyata Ikura, J. Organometal. Chem. 2, 146, (1964)] (16.4 g, 0.1 mmol) and sodium iodide (60 g, 0.4 mmol) in acetone (500 ml) are refluxed with stirring during 24 hours. The reaction mixture is cooled, filtered and the solvent is evaporated under reduced pressure. The residue is dissolved in ether and washed with water. The organic layer is dried over sodium sulfate, filtered and concentrated under reduced pressure to afford t-butyl(iodomethyl)dimethylsilane (20.9 g, 80%) as a slightly yellow oil.

EXAMPLE 13

Preparation of
5-AZIDO-3,6-DI-O-BENZYL-5-DEOXY-D-GLUCOFURANOSE

The azide 5-azido-3,6-di-O-benzyl-5-deoxy-1,2-O-isopropylidene-α-D-glucofuranoside (U.G. Nayak and R.L. Whisler, J. Org. Chem., 33, 3582 (1968) (15.02 g, 35.3 mmol) was dissolved at 0 °C in 100 ml of a 9:1 mixture of trifluoro-acetic acid and water. The mixture was stirred at 0 °C during 2 h. The trifluoroacetic acid was evaporated under reduced pressure at room temperature. The residue was taken with ether and washed with water. The organic layer was dried over sodium sulfate, filtered and concentrated under reduced pressure. Flash chromatography on silica gel and elution with a 1:1 mixture of hexane and ethyl acetate, followed by recrystallization in a mixture of hexane and ethyl acetate afforded the expected compound 5-azido-3,6-di-O-benzyl-5-deoxy-D-glucofuranose.

EXAMPLE 14

Preparation of
METHYL 5-AZIDO-3,6-DI-O-BENZYL-5-DEOXY-D-GLUCOFURANOSIDE

To a solution of 5-azido-3,6-di-O-benzyl-5-D-glucofuranose (10.23 g, 26.5 mmol) in methylene chloride (170 ml) was added methanol (11 ml) and borontrifluoroetherate (1.5 ml). The mixture was stirred 24 h at room temperature. The reaction mixture was successively washed with a saturated aqueous solution of sodium bicarbonate and then with brine. The organic layer was dried over sodium sulfate, filtered and concentrated under reduced pressure. Flash chromatography on silica gel and elution with a 1:1 mixture of hexane and ethyl acetate afforded methyl 5-azido-3,6-di-O-benzyl-5-deoxy-D-glucofuranoside as a colorless oil (9.15 g, 85%).

EXAMPLE 15

Preparation of
METHYL 5-AZIDO-2,3,6-TRI-O-BENZYL-5-DEOXY-D-GLUCOFURANOSIDE

To a suspension of sodium hydride (1.2 g, 27.5 mmol), 55% in mineral oil, washed three times with pentane) in anhydrous tetrahydrofuran (200 ml) was added quickly dropwise the alcohol methyl 5-azido-3,6-di-O-benzyl-5-deoxy-D-gluco-furanoside (9.15 g, 22.9 mmol) in tetrahydrofuran (50 ml) at room temperature and under nitrogen. The mixture was stirred during 3 h at room temperature. the mixture was yellow. Then $n\text{-}Bu_4N^+I^-$ (76 mg, 0.20 mmol) was added followed by benzyl bromide (3.30 ml, 27.5 mmol) added dropwise. The mixture was stirred overnight at room temperature. After hydrolysis with saturated aqueous ammonium chloride tetrahydrofuran was evaporated under reduced pressure. The residue was diluted with water and extracted three times with ether. The organic phase was dried over sodium sulfate. Filtration and evaporation under reduced pressure afforded an oil. Flash chromatography on silica gel and elution with a 20:80 mixture of ethyl acetate and hexane afforded the expected compound methyl 5-azido-2,3,6-tri-O-benzyl-5-deoxy-D-glucofuranoside as a colorless oil (10.88 g, 97%).

EXAMPLE 16

Preparation of
5-AZIDO-2,3,6-TRI-O-BENZYL-5-DEOXY-D-GLUCOFURANOSE

Methyl 5-azido-2,3,6-tri-O-benzyl-5-deoxy-D-glucofuranoside (10.8 g, 22.2 mmol) was dissolved at room temperature in tetrahydrofuran (20 ml). The solution was cooled at -10 °C and trifluoroacetic acid (120 ml) was added dropwise followed by addition of water (20 ml). The mixture was stirred at 0 °C during 24 h. The mixture was evaporated under reduced pressure without heating. The residue was taken with ether and washed with water. The organic layer was dried over sodium sulfate, filtered and concentrated under reduced pressure. Flash chromatography on silica gel and elution with a 20:80 mixture of ethyl acetate and hexane afforded 5-azido-2,3,6-tri-O-benzyl-5-deoxy-D-glucofuranose as a colorless oil (9.63 g, 90%).

EXAMPLE 17

Preparation of
5-AZIDO-2,3,6-TRI-O-BENZYL-5-DEOXY-D-GLUCONIC ACID-γ-LACTONE

To a solution of the lactol 5-azido-2,3,6-tri-O-benzyl-5-deoxy-D-glucofuranose (9.36 g, 20 mmol) in acetone (240 ml) cooled to 0 °C, Jones' reagent 2 M (11.5 ml) was added dropwise until the color was orange. The excess of Jones' reagent was destroyed with 2-propanol (0.5 ml). The mixture was concentrated under reduced pressure. The residue was taken with water and extracted with ether. The organic phase was dried with sodium sulfate, filtered and concentrated under reduced pressure so as to afford an oil. Flash chromatography on silica gel and elution with a 1:9 mixture of ethyl acetate and hexane afforded the γ-lactone 5-azido-2,3,6-tri-O-benzyl-5-deoxy-D-gluconic acid-γ-lactone.

EXAMPLE 18

Preparation of
2,3,6-TRI-O-BENZYL-5-DEOXY-D-GLUCONIC ACID-δ-LACTAM

To a solution of the lactone 5-azido-2,3,6-tri-O-benzyl-5-deoxy-D-gluconic acid-γ-lactone (8.16 g, 17 mmol) in ethanol (180 ml) was added lindlar catalyst (1.7 g). The mixture was hydrogenated under atmospheric pressure during 24 h. Filtration and evaporation under reduced pressure afforded an oil which was crystallized in a mixture of hexane and ether. The lactam 2,3,6-tri-O-benzyl-5-deoxy-D-gluconic acid-δ-lactam was obtained as white crystals (7.4 g, 96%). mp: 85-85.5° C.

EXAMPLE 19

Preparation of
2,3,6-TRI-O-BENZYL-1,5-DIDEOXY-1,5-IMINO-D-GLUCITOL

To a solution of 2,3,6-tri-O-benzyl-5-deoxy-D-gluconic acid δ-lactam (compound described in Example 18) (0.75 g, 1.6 mmol) in dry tetrahydrofuran (15ml) was added a 10 M solution of borane in methyl sulfide (0.58 ml) under nitrogen at 0° C. The mixture was stirred 15 min at 0° C, 30 min at room temperature, then refluxed during 6 h and finally stirred overnight at room temperature. The mixture was cooled to 0° C and the excess of borane was destroyed with methanol and stirred 1 h at room temperature. The reaction mixture was treated with gazeous hydrochloric acid and refluxed during 1 h. The solvents were evaporated under reduced pressure. The residue was dissolved in ethyl acetate and washed with a saturated aqueous solution of sodium bicarbonate. The organic layer was dried over sodium sulfate, filtered and concentrated under reduced pressure to afford an oil. Flash chromatography on silica gel and elution with ethyl acetate afforded 2,3,6-tri-O-benzyl-1,5-dideoxy-1,5-imino-D-glucitol which crystallized in methanol (0.655 g, 90%); m.p. 73-74° C.

EXAMPLE 20

Preparation of
1,5-DIDEOXY-1,5-{[4-(TRIMETHYLSILYL)-2-BUTYNYL]IMINO}-D-GLUCITOL

A solution of 1,5-dideoxy-1,5-imino-D-glucitol (0.5 g, 3.06 mmol) and (4-bromo-2-butynyl)trimethylsilane (0.943 g, 4.6 mmol) is dissolved in dimethylformamide (15 ml) containing water (0.5 ml). Triethylamine (0.85 ml) is added. The mixture is heated at 80° C during 24 hours. The solvent is evaporated under reduced pressure. Flash chromatography on silica gel and elution with a 8:2 mixture of chloroform and methanol affords 1,5-dideoxy-1,5-{[4-(trimethylsilyl)-2-butynyl]imino}-D-glucitol as an amorphous solid (0.24 g, 27%).

EXAMPLE 21

Preparation of
1,5-DIDEOXY-1,5-{[4-(TRIMETHYLSILYL)-2(Z)-BUTENYL]IMINO}-D-GLUCITOL

1,5-Dideoxy-1,5-{[4-(trimethylsilyl)-2-butynyl]imino}-D-glucitol (0.1 g, 0.35 mmol) is dissolved in methanol (5 ml) and lindlar catalyst (25 mg) is added. The mixture is hydrogenated at atmospheric pressure overnight. The catalyst is filtered off and the solvent is evaporated under reduced pressure to afford 1,5-dideoxy-1,5-{[4-(trimethylsilyl)-2(Z)-butenyl]imino}-D-glucitol as an amorphous solid (0.09 g, 90%).

EXAMPLE 22

Preparation of
1,5-DIDEOXY-1,5-{[4-(TRIMETHYLSILYL)-2(E)-BUTENYL]IMINO}-D-GLUCITOL

A solution of 1,5-dideoxy-1,5-imino-D-glucitol (0.5 g, 3.06 mmol) and (4-bromo-2-(E)butenyl)-trimethylsilane (0.95 g, 4.6 mmol) in a mixture of dimethylformamide (10 ml), water (0.5 ml) and triethylamine (0.85 ml) is heated at 80° C during 24 hours. The solvents are evaporated under reduced pressure. Flash chromatography on silica gel and elution with a 8:2 mixture of chloroform and methanol affords 1,5-dideoxy-1,5-{[4-(trimethylsilyl)-2(E)-butenyl]imino}-D-glucitol as a foam (0.12 g, 14%).

EXAMPLE 23

Preparation of

1,5-DIDEOXY-2,3,6-TRI-O-BENZYL-1,5-{[3-(DIMETHYLPHENYLSILYL)PROPYL]IMINO}-D-GLUCITOL

A solution of 1,5-dideoxy-2,3,6-tri-O-benzyl-1,5-imino}-D-glucitol(0.433 g, 1 mmol) and dimethyl(3-iodopropyl)phenylsilane (0.912 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-o-benzyl-1,5-{[3-(dimethylphenylsilyl)propyl]imino}-D-glucitol as a colorless oil (0.493 g, 81%).

EXAMPLE 24

Preparation of
1,5-DIDEOXY-1,5-{[3-(DIMETHYLPHENYLSILYL)PROPYL]IMINO}-D-GLUCITOL

1,5-Dideoxy-2,3,6-tri-O-benzyl-1,5-{[3-(dimethylphenylsilyl)propyl]imino}-D-glucitol (0.45 g, 0.74 mmol) is dissolved in a 9:1 mixture of methanol and formic acid (10 ml), and palladium 10% on charcoal (0.45 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$. Water is evaporated under reduced pressure and flash chromatography on silica gel and elution with a 8:2 mixture of chloroform and methanol affords the expected product 1,5-dideoxy-1,5-{[3-(dimethylphenylsilyl)propyl]imino}-D-glucitol as an amorphous solid (0.208 g, 83%).

EXAMPLE 25

Preparation of
1,5-DIDEOXY-2,3,6-TRI-O-BENZYL-1,5-{[(BENZYLDIMETHYLSILYL)METHYL] IMINO}-D-GLUCITOL

A solution of 1,5-dideoxy-2,3,6-tri-O-benzyl-1,5-imino-D-glucitol (0.433 g, 1 mmol) and benzyl-(iodomethyl)dimethylsilane (0.87 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-bensyl-1,5-{[(bensyldimethylsilyl)-methyl]imino}-D-glucitol as a colorless oil (0.386 g, 65%).

EXAMPLE 26

Preparation of
1,5-DIDEOXY-1,5-{[(BENZYLDIMETHYLSILYL)METHYL]IMINO}-D-GLUCITOL

1,5-Dideoxy-2,3,6-tri-O-benzyl-1,5-{[(benzyldimethylsllyl)methyl]imino]-D-glucitol (0.3 g, 0.5 mmol) is dissolved in a 9:1 mixture of methanol and formic acid (10 ml), and palladium 10% on charcoal (0.3 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$. Water is evaporated under reduced pressure and flash chromatography on silica gel and elution with a 8:2 mixture of chloroform and methanol affords the expected product 1,5-dideoxy-1,5-{[(bensyldimethylsilyl)-methyl]-imino}-D-glucitol as an amorphous solid (0.09 g, 55%).

EXAMPLE 27

Preparation of
1,5-DIDEOXY-2,3,6-TRI-O-BENZYL-1,5-{[(t-BUTYLDIMETHYLSILYL)METHYL]IMINO]-D-GLUCITOL

A solution of 1,5-dideoxy-2,3,6-tri-O-benzyl-1,5-imino-D-glucitol (0.433 g, 1 mmol) and t-butyl-(iodomethyl)dimethylsilane (0.77 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate,

filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[(t-butyldimethylsilyl)methyl]imino}-D-glucitol as a colorless oil (0.42 g, 75%).

EXAMPLE 28

Preparation of
1,5-DIDEOXY-1,5-{[(t-BUTYLDIMETHYLSILYL)METHYL]IMINO}-D-GLUCITOL

1,5-Dideoxy-2,3,6-tri-O-benzyl-1,5-{[(t-butyldimethylsilyl)methyl]imino}-D-glucitol (0.4 g, 0.72 mmol) is dissolved in a 9:1 mixture of methanol and formic acid (10 ml), and palladium 10% on charcoal (0.5 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$. Water is evaporated under reduced pressure and flash chromatography on silica gel and elution with a 8:2 mixture of chloroform and methanol affords the expected product 1,5-dideoxy-1,5-{[(t-butyldimethylsilyl)methyl]imino}-D-glucitol as an amorphous solid (0.127 g, 61%).

EXAMPLE 29

Preparation of
1,5-DIDEOXY-2,3,6-TRI-O-BENZYL-1,5-{[(DIMETHYLPHENYLSILYL)METHYL]IMINO}-D-GLUCITOL

A solution of 1,5-dideoxy-2,3,6-tri-O-bensyl-1,5-imino-D-glucitol (0.433 g, 1 mmol) and phenyl-(iodomethyl)dimethylsilane [Chih-Tang Huang and Pao-Jen Wang, Hua Hsüeh Hsüeh Pao, 25, 341, (1959)] (0.77 g, 3 mool) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-bensyl-1,5-{[(dimethylphenylsilyl)methyl]imino}-D-glucitol as a colorless oil(0.37g, 64%).

EXAMPLE 30

Preparation of
1,5-DIDEOXY-1,5-{[(DIMETHYLPHENYLSILYL)METHYL]IMINO}-D-GLUCITOL

1,5-Dideoxy-2,3,6-tri-O-benzyl-1,5-{[(dimethylphenylsilyl)-methyl]imino}-D-glucitol (0.35 g, 0.60 mmol) is dissolved in a 9:1 mixture of methanol and formic acid (10 ml), and palladium 10% on charcoal (0.35 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$. Water is evaporated under reduced pressure and flash chromatography on silica gel and elution with a 8:2 mixture of chloroform and methanol affords the expected product 1,5-dideoxy-1,5-{[(dimethylphenylsilyl)-methyl]imino}-D-glucitol as an amorphous solid (0.139 g, 75%).

EXAMPLE 31

Preparation of
1,5-DIDEOXY-2,3,6-TRI-O-BENZYL-1,5-{[(TRIMETHYLSILYL)METHYL]IMINO}-D-GLUCITOL

A solution of 1,5-dideoxy-2,3,6-tri-O-benzyl-1,5-imino-D-glucitol (0.1 g, 0.24 mmol) and (iodomethyl)-trimethylsilane (0.45 ml, 3.1 mmol) in a mixture of dimethylformamide (3 ml) and triethylamine (0.44 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[(trimethylsilyl)methyl]imino}-D-glucitol as a colorless oil(0.09g, 75%).

EXAMPLE 32

Preparation of
1,5-DIDEOXY-1,5-{[(TRIMETHYLSILYL)METHYL]IMINO}-D-GLUCITOL

1,5-Dideoxy-2,3,6-tri-O-benzyl-1,5-{[(trimethylsilyl)methyl]imino}-D-glucitol (0.075 g, 0.14 mmol) is dissolved in a 9:1 mixture of methanol and formic acid (12 ml), and palladium 10% on charcoal (0.3 g) is added. The mixture is stirred overnight at room temperature. The catalyst is removed by filtration. The solvents are evaporated under reduced pressure. The residue is dissolved in water and neutralized with AG1-X8, 20-50 mesh, $OH^{\ominus}$ form. The resin is removed by filtration, water is removed by lyophilization and the expected product 1,5-dideoxy-1,5-{[(trimethylsilyl)methyl]imino}-D-glucitol is obtained as an amorphous solid (0.016 g, 45%).

EXAMPLE 33

Preparation of
1,5-DIDEOXY-2,3,6-TRI-O-BENZYL-1,5-{[3-(TRIMETHYLSILYL)PROPYL]IMINO)-D-GLUCITOL

A solution of 1,5-dideoxy-2,3,6-tri-O-benzyl-1,5-imino-D-glucitol (0.34 g, 0.78 mmol) and (3-iodopropyl)-trimethylsilane (0.57 gr, 2.34 mmol) in a mixture of dimethylformamide (5 ml) and triethylamine (0.33 ml) is stirred at room temperature during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[3-(trimethylsilyl)-propyl]imino}-D-glucitol as a colorless oil(0.405g, 94%).

EXAMPLE 34

Preparation of
1,5-DIDEOXY-1,5-{[3-(TRIMETHYLSILYL)PROPYL]IMINO}-D-GLUCITOL

1,5-Dideoxy-2,3,6-tri-O-benzyl-1,5-{[3-(trimethylsilyl)propyl]imino}-D-glucitol (0.39 g, 0.7 mmol) is dissolved in a 9:1 mixture of methanol and formic acid (30 ml), and palladium 10% on charcoal (2 g) is added. The mixture is stirred overnight at room temperature. The catalyst is removed by filtration. The solvents are evaporated under reduced pressure. The residue is dissolved in water and neutralized with AG1-X8, 20-50 mesh, $OH^{\ominus}$ form. The resin is removed by filtration, water is removed by lyophilization and the expected product 1,5-dideoxy-1,5-{[(trimethylsilyl)propyl]imino}-D-glucitol is obtained as an amorphous solid (0.17 g, 85%).

EXAMPLE 35

Preparation of
1,5-DIDEOXY-2,3,6-TRI-O-BENZYL-1,5-{[4-(TRIMETHYLSILYL)BUTYL]IMINO}-D-GLUCITOL

A solution of 1,5-dideoxy-2,3,6-tri-O-benzyl-1,5-imino-D-glucitol (0.043 g, 0.1 mmol) and (4-iodobutyl)-trimethylsilane (0.088 g, 0.3 mmol) in a mixture of dimethylformamide (0.8 ml) and triethylamine (0.04 ml) is stirred at room temperature during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[4-(trimethylsilyl)butyl]imino}-D-glucitol as a colorless oil (0.05 g, 90%).

EXAMPLE 36

Preparation of
1,5-DIDEOXY-1,5-{[4-(TRIMETHYLSILYL)BUTYL]IMINO}-D-GLUCITOL

1,5-Dideoxy-2,3,6-tri-O-benzyl-1,5-{[4-(trimethylsilyl)butyl]imino}-D-glucitol (0.05 g, 0.09 mmol) is dissolved in a 9:1 mixture of methanol and formic acid (15 ml), and palladium 10% on charcoal (0.2 g) is added. The mixture is stirred overnight at room temperature. The catalyst is removed by filtration. The

solvents are evaporated under reduced pressure. The residue is dissolved in water and neutralized with AG1-X8, 20-50 mesh, $OH^{\ominus}$ form. The resin is removed by filtration, water is removed by lyophilization and the expected product 1,5-dideoxy-1,5-{[4-(trimethylsilyl)butyl]imino}-D-glucitol is obtained as an amorphous solid (0.02 g, 75%).

Enzymes which catalyze the hydrolysis of complex carbohydrates, e.g. α-glycosidases, convert non-absorbable carbohydrates into absorbable sugars. The rapid action of these enzymes, particularly following the intake of high levels of carbohydrates, lead to acute high levels in blood glucose which, in the case diabetics, lead to undesirable manifestations, thus it has been a long-sought goal to find compounds which will obviate the hyperglicemia caused by dietary improprieties. Similarly, in the case of obesity the control of high levels of blood glucose, with its subsequent conversion to fat, caused by the catalysis of carbohydrates has inspired the quest for compounds which will obviate the problems associated with dietary improprieties.

The compounds of this invention (I) are potent and long-lasting inhibitors of α-glucosidase and, by standard laboratory methods for determining serum glucose levels, are shown to be useful for the treatment of disease states caused by the underutilization and/or overproduction of serum glucose without adversely affecting the rate of transport across cell membranes. Thus, the compounds are useful in the treatment of diabetes and obesity.

In the practice of this invention, an effective amount of a compound of this invention is that amount required to reduce the amount of serum glucose (relative to a control) following the ingestion of carbohydrates convertible to absorbable glucose. The specific dosage for the treatment of any specific patient suffering from either disease state will depend upon such factors as size, type and age of the patient as well as the patients' dietary habits and the severity of the disease state, all of which are factors normally familiar to and considered by the attending diagnostician treating the patient. Generally, the compounds are to be administered orally at a dose of 0.01 to 2 milligrams per kilogram of body weight (MPK) with a dose of 0.025 to 0.5 MPK being preferred. The compounds preferably are to be administered orally at mealtimes in single or multiple unit doses containing 1 mg to 10 mg. Of course, in the treatment of obesity, the term includes the practice of the disease as well as continuel administration of dose regimens suitable for the maintenance of the desired weight for the patient.

It is also to be found that the compounds of the instant invention (I) will exert an inhibitory effect on glycosidase enzymes that are essential for elaboration of the final structure of the oligosaccharide side-chains of glyco-proteins, particularly the HIV (gp 120) glycoprotein. Suitable assay techniques, e.g. syncytial formation, the reverse transcriptase assay, immunofluorescence tests and election microscopy, may be used to evaluate the effects on HIV viral growth and for determining dose regimens. Antiviral effects may be confirmed by immunofluorescence with serum for virally infected patients. In the treatment of the HIV related disease states, as well as other retroviral glyco-protein-related disease states, unlike the treatment of diabetes and obesity, the compounds of this invention may be administered by parenteral means; specific doses being within the above stated dose range for treatment of diabetes and obesity. The compounds of this invention may also be effectively utilized in conjunctive therapy with compounds known to be useful in treating patients with AIDS such as for example 2,3-dideoxycytidine, 2,3-dideoxyadenine, interferon, interleukin-2 and the like.

In practising the end-use application of the compounds of this invention, the compounds are preferably incorporated in a pharmaceutical formulation comprising a pharmaceutical carrier in admixture with a compound of this invention. The term "pharmaceutical carrier" refers to known pharmaceutical excipients useful in formulating pharmaceutically active compounds for internal administration to animals, and which are substantially non-toxic and non-sensitizing under conditions of use. The compositions can be prepared by known techniques for the preparation of tablets, capsules, elixirs, syrups emulsions, dispersions and wettable and effervescent powders, and can contain suitable excipients known to be useful in the preparation of the particular type of composition desired. Suitable pharmaceutical carriers and formulation techniques are found in standard texts, such as Remington's Pharmaceutical Sciences, Mack Publishing Company, Easton, Pa.

Particularly preferred compounds are those wherein:

Q is methyl, n-propyl, n-butyl, allyl, propargyl, and butenyl-2,

$R_1$ is methyl and phenyl,

$R_2$ and $R_3$ are methyl and/or phenyl or benzyl,

with the following specific compounds being most preferred:

1,5-dideoxy-1,5-{[(trimethylsilyl)methyl]imino}-D-glucitol,

1,5-dideoxy-1,5-{[(dimethylphenylsilyl)methyl]imino}-D-glucitol,

1,5-dideoxy-1,5-{[3-(trimethylsilyl)propyl]imino}-D-glucitol,

1,5-dideoxy-1,5-{[4-(trimethylsilyl)butyl]imino}-D-glucitol,
1,5-dideoxy-1,5-{[4-(trimethylsilyl)-2-butenyl]imino}-D-glucitol,

## Claims

1. A compound of the formula

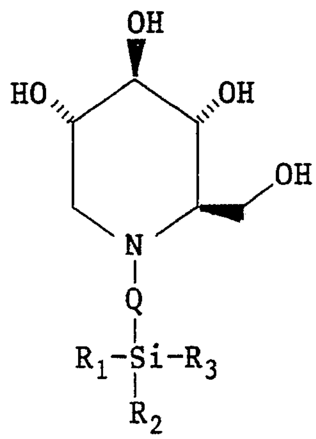

I

and the pharmaceutically acceptable salts thereof wherein

Q is $C_{1-7}$ alkylene, $-(CH_2)_m-HC=CH-(CH_2)_n$, $-(CH_2)_mC\equiv C-(CH_2)_n$,$(CH_2)_mCH=C=CH(CH_2)_n$ with m being 1 or 2 and n being zero, 1 or 2,

$R_1$ is $C_{1-7}$ alkyl or $C_{1-7}$ alkoxy, each of

$R_2$ and $R_3$ are $C_{1-10}$ alkyl or $-(CH_2)_p$-X,Y-substituted phenyl, with X and Y each being H, OH, halogeno, $C_{1-6}$ alkyl, $C_{1-6}$ alkoxy, $CF_3$, CN, $NO_2$, SH or $-S-C_{1-6}$ alkyl, and p is an integer 1 to 4 or zero.

2. The compound according to claim 1 for use as pharmaceutically active agent.

European Patent Office

# EUROPEAN SEARCH REPORT

Application Number

**EP 90 40 1169**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 350 012 (MEIJI SEIKA KAISHA LTD)<br>* Whole document *<br>– – – | 1-2 | C 07 F 7/08<br>A 61 K 31/695<br>C 07 F 7/18 |
| A | EP-A-0 282 618 (VERENIGING HET NEDERLANDS KANKER INSTITUUT)<br>* Claim 1 *<br>– – – – – | 2 | |

TECHNICAL FIELDS SEARCHED (Int. Cl.5)

C 07 F 7/00
A 61 K 31/00

The present search report has been drawn up for all claims

| Place of search | Date of completion of search | Examiner |
|---|---|---|
| The Hague | 14 November 90 | RINKEL L.J. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document